# EUROPEAN PATENT APPLICATION

(11) **EP 4 325 223 A2**
(43) Date of publication of application: **21.02.2024**
(21) Application number: 23205252.2
(22) Date of filing: 23.12.2020
(51) Int. Cl.: G01N 33/68

(54) **PROTEIN AND PEPTIDE FINGERPRINTING AND SEQUENCING BY NANOPORE TRANSLOCATION OF PEPTIDE-OLIGONUCLEOTIDE COMPLEXES**

(30) Priority: 24.12.2019 NL 2024579
(62) Divisional of application: 20830378.4
(71) Applicant: Technische Universiteit Delft, 2628 CN Delft (NL)
(72) Inventor: BRINKERHOFF, Henry Derek, 2600 AA Delft (NL); DEKKER, Cornelis, 2600 AA Delft (NL)
(74) Representative: J A Kemp LLP

(57) **Abstract**

A method (100) for translocation of a peptide (11) through a nanopore (50) wherein the method (100) comprises translocating the peptide (11) in the presence of an oligonucleotide translocase (20), wherein the peptide (11) is comprised by a peptideoligonucleotide complex (10), wherein the peptide (11) is linked to an oligonucleotide (13), wherein the oligonucleotide translocase (20) is associated to the oligonucleotide (13) during at least part of the translocation.

## Description

### FIELD OF THE INVENTION

The invention relates to a method for translocating a peptide through a nanopore. The invention further relates to an analysis method for analyzing a peptide. The invention further relates to the use of an oligonucleotide translocase to translocate a peptide-oligonucleotide complex. The invention further relates to the use of a nanopore for characterizing a peptide-oligonucleotide complex. The invention further relates to the use of a nanopore for fingerprinting or sequencing a protein. The invention further relates to the use of a nanopore for characterizing posttranslational modifications on a protein. The invention further relates to a kit of parts.

### BACKGROUND OF THE INVENTION

Methods for translocating a peptide through a nanopore are known in the art. For example, WO2013123379A2 describes a device and method for translocating a protein through a nanopore and monitoring electronic changes caused by different amino acids in the protein. The device comprises a nanopore in a membrane, an amplifier for providing a voltage between the cis side and trans side of the membrane, and an NTP driven unfoldase which processed the protein to be translocated. The exemplified unfoldase is the ClpX unfoldase from E. coli.

US20170199149 describes a method for nanopore-based protein analysis. The method addresses the characterization of a target protein analyte, which has a dimension greater than an internal diameter of the nanopore tunnel, and which is also physically associated with a polymer. The methods further comprises applying an electrical potential to the nanopore system to cause the polymer to interact with the nanopore tunnel. The ion current through the nanopore is measured to provide a current pattern reflective of the structure of the portion of the polymer interacting with the nanopore tunnel. This is used as a metric for characterizing the associated protein that does not pass through the nanopore.

Nivala, et al., "Unfoldase-mediated protein translocation through an α-hemolysin nanopore", Nature Biotechnology, 2013 describes unfolding and translocation of proteins through the a-hemolysin pore using the AAA+ unfoldase CIpX. It further describes that sequence-dependent features of individual engineered proteins were detected during translocation.

WO2012107778 describes mutant forms of Msp porins. It further describes nucleic acid characterisation using Msp. Specifically, it describes a method of characterising a target nucleic acid sequence, comprising: (a) contacting the target sequence with an Msp-derived pore and a nucleic acid binding protein so that the protein controls the movement of the target sequence through the pore and a proportion of the nucleotides in the target sequence interacts with the pore; and (b) measuring the current passing through the pore during each interaction and thereby characterising the target sequence.

WO2017125565 describes a method for preparing nanopore sequencing complexes in membranes for sequencing of polymers, e.g., polynucleotides and polypeptides. The nanopore sequencing complex is formed by the sequential linking of an enzyme to a nanopore that is inserted in a membrane, and of a polymer to the enzyme. Alternatively, the nanopore sequencing complex is formed by linking a preformed enzyme-polymer complex to a nanopore that is inserted in a membrane.

Derrington, et al., "Subangstrom single-molecule measurements of motor proteins using a nanopore", Nature Biotechnology, 2015, describes a method for monitoring the motion of DNA and conformational changes of processive nucleic-acid-binding proteins as the nucleic acid passes through the nanopore.

### SUMMARY OF THE INVENTION

The central dogma of biology may hold that DNA encodes the information required to create proteins, which perform many of the functions we associate with living organisms. Proteins catalyze reactions, transport chemicals, send and carry signals, create structure, and more. To create a protein, a gene may be transcribed from DNA onto messenger RNA, and the RNA may then be translated into the chain of amino acids to provide the protein. Despite the important roles proteins play in living organisms, the detection and characterization of proteins may remain a costly and time-consuming process.

Protein detection and characterization by mass spectrometry, which may be the current gold standard, may require extensive sample preparation and large sample sizes, while possessing a limited dynamic range with respect to sample concentration. These limitations may severely restrict the application of mass spectrometry-based protein characterization to biological and clinical problems. Further, mass spectrometry may rely on ionization and fragmentation of the proteins, and subsequent detection of the masses of the protein fragments. Based on the detected masses and their frequencies, the identity and abundance of proteins may be inferred based on masses corresponding to protein sequences predicted from genetic information. However, the process may be limited as (i) different potential fragmentation patterns may exist for a protein, (ii) post-translationally modified proteins may be difficult to characterize, particularly as an exhaustive overview of potential post-translational modifications for a given protein may typically not be available, (iii) low abundance proteins may be hard to detect, and/or (iv) there may be an information loss due to protein fragmentation, as similar proteins may provide (some) identical protein fragments, which may be particularly problematic with regards to protein isoforms.

Immunoassays for protein detection and characterization may only be capable of analyzing relatively few proteins in a sample. Further, immunoassays may be time-intensive to adapt and may be limited by the availability of antibodies, which may also often present specificity issues.

Nanopore-based detection and characterization of a molecule may be based on imposing a potential difference over a membrane comprising a nanopore and detecting changes in the electrical current through the nanopore as the molecule passes through the nanopore and provides transient blockages. Thereby, a nanopore may be used to differentiate molecules, including proteins, as they transiently pass through the nanopore, based on the dwell time, ion current structure, or noise spectrum of these transient blockages. The prior art may describe the use of an unfoldase protein to translocate a to-be-analyzed protein through a nanopore. However, the steps of the unfoldase along the amino acid chain of the protein may be irregular in frequency and size, and the unfolding of the protein by the unfoldase may lead to additional granularity and uncertainty in the position of the protein within the pore.

Hence, it is an aspect of the invention to provide an alternative method for the detection and characterization of proteins, which preferably further at least partly obviates one or more of above-described drawbacks. The present invention may have as object to overcome or ameliorate at least one of the disadvantages of the prior art, or to provide a useful alternative.

Hence, in a first aspect the invention may provide a method for the translocation of a peptide through a nanopore. The method may especially comprise translocating the peptide (through the nanopore) in the presence of an oligonucleotide translocase. The peptide may especially be comprised by a peptide-oligonucleotide complex (also "complex" or "oligonucleotide-peptide complex"), especially wherein the peptide is linked to an oligonucleotide. In embodiments, the oligonucleotide translocase may be associated to the oligonucleotide during at least part of the translocating. In further embodiments, the oligonucleotide translocase may affect the translocating of the peptide-oligonucleotide complex through the nanopore. In particular, the method may comprise translocating at least part of the peptide-oligonucleotide complex through the nanopore with the oligonucleotide translocase, *i.e.,* the oligonucleotide translocase may translocate at least part of the peptide-oligonucleotide complex through the nanopore.

In specific embodiments, the invention may provide a method for the translocation of a peptide through a nanopore in the presence of an oligonucleotide translocase, wherein the peptide is comprised by a peptide-oligonucleotide complex, wherein the peptide is linked to an oligonucleotide, and wherein the oligonucleotide translocase is associated to the oligonucleotide during at least part of the translocating.

The method of the invention may provide the benefit that the peptide-oligonucleotide complex can be translocated through the nanopore by an oligonucleotide translocase. Oligonucleotide translocases may provide one or more of: (i) generally translocating the oligonucleotide in one direction, (ii) generally taking steps of a regular (or "consistent") size, and (iii) stepping at a desirable rate. Thereby, the peptide may be translocated through the nanopore in a manner suitable for detecting and characterizing of the peptide.

The method of the invention may enable a single-molecule protein sequencer, in which ion current measurements are taken using a hybrid of a peptide fragment. For the combination of the nanopore protein MspA, which may previously have been shown suitable for DNA sequencing, and the oligonucleotide translocase Hel308, the separation between an anchor site for the oligonucleotide translocase and the nanopore constriction may be about 9 nm (also see further below), which may correspond to an amino acid read length of about 26 amino acids. The ion current measurements, especially the ion current profile, of a (or: "corresponding to") peptide may be used, for example, to (i) sequence the peptide (i.e., determine the (sequence of the) constituent amino acids), (ii) to identify the peptide from a library of proteins or peptides, and/or (iii) to map variant sites such as post-translational modifications.

The method may be particularly suited for detecting and characterizing peptides from biological systems, such as in the context of (medical) research, or in the context of diagnostics. The method may further be suitable for the detecting and characterizing of proteins by first fragmenting the proteins into peptides through, for example, enzymatic or chemical digestion. The method may especially be for (at least partially) translocating (also "passing") a peptide through a nanopore in the presence of an oligonucleotide translocase. Hence, in embodiments, essentially the entire peptide may translocate through the nanopore in the presence of the oligonucleotide translocase, whereas in other embodiments the peptide may partially translocate through the nanopore in the presence of the oligonucleotide translocase. In further embodiments, the oligonucleotide translocase may affect (or "modulate") the translocation of the peptide through the nanopore. In particular, the oligonucleotide translocase may push or pull (at least part of) the peptide through the nanopore (during at least part of the translocation).

Hence, in embodiments, the oligonucleotide translocase may translocate the peptide through the nanopore, especially by processing the oligonucleotide, more especially by generating a complementary strand of the oligonucleotide.

The phrase "the oligonucleotide translocase is associated to the oligonucleotide" and similar phrases may herein especially refer to the oligonucleotide translocase being functionally coupled to the oligonucleotide, especially being bound to the oligonucleotide.

In embodiments, the method may comprise a translocation stage. The translocation stage may especially comprise translocating the peptide (through the nanopore) in the presence of an oligonucleotide translocase. In particular, the oligonucleotide translocase may associate with the oligonucleotide during at least part of the translocation stage. In further embodiments, the oligonucleotide translocase may affect the translocating of the peptide-oligonucleotide complex through the nanopore during at least part of the translocation stage. In particular, the translocation stage may comprise translocating at least part of the peptide-oligonucleotide complex through the nanopore with the oligonucleotide translocase, *i.e.,* the oligonucleotide translocase may translocate at least part of the peptide-oligonucleotide complex through the nanopore during at least part of the translocation stage.

The phrase "translocating through the nanopore" and similar phrases may herein especially refer to an element, such as a single amino acid in the amino acid chain of a peptide, passing the constriction of the nanopore. The constriction may especially be a cross-section within the nanopore with the smallest (also: narrowest) circular equivalent constriction diameter, wherein the cross-section is perpendicular to the longitudinal dimension (*i.e.,* from one end of the nanopore to the other end) of the nanopore.

In embodiments, the peptide-oligonucleotide complex may at least partially be translocated through the nanopore due to a potential difference imposed over the nanopore. The phrase "a potential difference imposed over the nanopore" may herein especially refer to a potential difference being imposed between a cis side of the nanopore and a trans side of the nanopore.

The term "peptide" may refer herein to a chain of amino acids of any length, especially a chain of at least two amino acids, more especially a chain of five or more amino acids. The term peptide may thus also refer to a protein, oligopeptide and polypeptide. In embodiments, the peptide may comprise a number of amino acids selected from the range of 2-60 amino acids. In further embodiments, the peptide may have at most 50 amino acids, such as at most 40 amino acids, especially at most 35 amino acids, such as at most 30 amino acids, especially at most 25 amino acids, such as at most 20 amino acids. The term "peptide" may herein also refer to a plurality of (different) peptides.

As the nanopore may be exposed to a single peptide-oligonucleotide complex at a time, the method may be (essentially) unhampered by a mixture of different peptides, especially by a mixture of different peptide-oligonucleotide complexes (comprising different peptides).

The oligonucleotide translocase may especially be an enzyme configured to process an oligonucleotide, wherein the oligonucleotide translocase and the oligonucleotide move relative to one another as the oligonucleotide translocase processes the oligonucleotide. In embodiments, the oligonucleotide translocase may comprise an enzyme selected from the group comprising a helicase, a polymerase, and a reverse transcriptase, especially from the group comprising a helicase and a polymerase, especially a helicase, or especially a (DNA) polymerase.

In embodiments, the peptide may be comprised by a peptide-oligonucleotide complex, *i.e.,* a peptide-oligonucleotide complex may comprise the peptide.

In further embodiments, the peptide may be linked to an oligonucleotide. Hence, the peptide-oligonucleotide complex may comprise the peptide and the oligonucleotide, especially wherein the peptide and the oligonucleotide are linked.

The term "oligonucleotide" may herein refer to a chain of nucleotides of any length, especially a chain of at least two nucleotides, more especially a chain of five or more nucleotides. In embodiments, the oligonucleotide may comprise at least 10 nucleotides, such as at least 15 nucleotides, especially at least 20 nucleotides. In further embodiments, the oligonucleotide may comprise at most 1000 nucleotides, such as at most 500 nucleotides, especially at most 100 nucleotides, such as at most 50 nucleotides, especially at most 40 nucleotides. During part of the method, the oligonucleotide may be (at least partially) bound to a complementary oligonucleotide.

In embodiments the oligonucleotide may comprise a DNA oligonucleotide, especially a DNA oligonucleotide consisting of canonical nucleobases. DNA oligonucleotides may be preferred as DNA-translocases may be particularly suitable as they may be more consistent in step-size and step-frequency when compared to other oligonucleotide translocases.

In further embodiments, the oligonucleotide may comprise an RNA oligonucleotide. In further embodiments, the oligonucleotide may comprise an XNA (xenonucleic acid) oligonucleotide. In further embodiments, the XNA oligonucleotide may be a PNA (peptide nucleic acid) oligonucleotide. In further embodiments, the oligonucleotide may comprise a DNA oligonucleotide comprising non-canonical nucleobases, such as, for example, the complimentary nucleosides dNaM and d5-SICS. d5-SICS may be an artificial nucleoside comprising a 6-methylisoquinoline-1-thione-2-yl group instead of a base. dNaM may be an artificial nucleoside comprising a 3-methoxy-2-naphthyl group instead of a base.

In further embodiments, the complimentary oligonucleotide may comprise an oligonucleotide selected from the group comprising a DNA oligonucleotide, an RNA nucleotide and an XNA oligonucleotide, especially from the group comprising a DNA oligonucleotide and an RNA nucleotide. In further embodiments, the oligonucleotide and the complementary oligonucleotide may be the same type of oligonucleotide, e.g., both DNA oligonucleotides. In further embodiments, the oligonucleotide and the complimentary oligonucleotide may be a different type of oligonucleotide, e.g., a DNA oligonucleotide and an RNA oligonucleotide.

The peptide-oligonucleotide complex may further comprise a linker arranged between the peptide and the oligonucleotide, *i.e.,* the peptide and the oligonucleotide may be linked via a linker (see further below).

In embodiments, the oligonucleotide translocase may (be configured to) affect the translocating of the peptide-oligonucleotide complex through the nanopore.

In further embodiments, the method may comprise translocating at least part of the peptide-oligonucleotide complex through the nanopore with the oligonucleotide translocase. In particular, the oligonucleotide translocase may be configured to translocate at least part of the peptide-oligonucleotide complex through the nanopore. Especially, the oligonucleotide translocase may (actively) move the oligonucleotide, thereby pulling (or pushing) the peptide-oligonucleotide complex through the nanopore.

In further embodiments, the peptide-oligonucleotide complex may translocate through the nanopore due to a potential difference imposed over the nanopore, wherein the oligonucleotide translocase associates with, especially binds, the oligonucleotide, and wherein during at least part of the method the oligonucleotide translocase reduces the translocation rate of the peptide-oligonucleotide complex, especially by acting as a brake. Essentially, in such embodiments, the oligonucleotide may be "pulled through" the nanopore and the oligonucleotide translocase due to the imposed potential difference, wherein the oligonucleotide translocase reduces the rate of translocation of the protein through the nanopore.

In embodiments, the nanopore may comprise a constriction. The nanopore constriction may be the space within the nanopore with the smallest (also: narrowest) circular equivalent diameter d_{c}, especially the smallest circular equivalent diameter d_{c} essentially perpendicular to a longitudinal direction of the nanopore. The presence of a (part of a) molecule at the constriction may provide the largest modulations to the ionic current.

In further embodiments, the constriction may have a circular equivalent constriction diameter d_{c}. In further embodiments, the circular equivalent constriction diameter d_{c} may be ≤ 5 nm, especially ≤ 4 nm, such as ≤ 3.5 nm, especially ≤ 3 nm, such as ≤ 2.5 nm, especially ≤ 2 nm. In further embodiments, the constriction diameter may be ≥ 0.1 nm, especially ≥ 0.2 nm, such as ≥ 0.3 nm, especially ≥ 0.5 nm, such as ≥ 1 nm, especially ≥ 1.5 nm, such as ≥ 2 nm.

In embodiments, the nanopore may comprise a solid state nanopore, especially a solid state nanopore in a synthetic membrane. Essentially, the solid state nanopore may be formed by providing a hole in the synthetic membrane, via, for example, one or more of ion beam sculpting and/or transmission electron microscopy. The nanopore may especially be arranged such that a molecule, such as the peptide, could pass from a cis side of the synthetic membrane via the nanopore to a trans side of the membrane. In embodiments, the synthetic membrane may comprise a material selected from the group comprising silicon nitride (SiN), graphene, especially box-shaped graphene, molybdenum disulfide (MoS₂), boron nitride (BN), and silicon dioxide (SiO₂).

In embodiments, the nanopore may be comprised by or may be formed by a nanopore protein. The nanopore protein may be selected from the group comprising MspA, CgG, FraC, ClyA, especially MspA. In further embodiments, the nanopore protein may comprise a mutant of a protein selected from the group comprising MspA, CgG, FraC, ClyA, especially MspA.

In further embodiments, the nanopore protein may comprise an MspA mutant, especially M2-NNN-MspA, described by Butler, "Single-molecule DNA detection with an engineered MspA protein nanopore", PNAS, volume 105, 2008, which is hereby incorporated herein by reference. M2-NNN-MspA may herein especially refer to the D90N/D91N/D93N/D118R/E139K/D134R MspA mutant, wherein the indicated amino acid substitutions are relative to MspA corresponding to NCBI Accession Number CAB56052.1. In particular, M2-NNN-MspA may comprise mutation to reduce negative charges at the constriction to facilitate the passage of an oligonucleotide. However, in further embodiments, the MspA mutant may comprise an increased number of negative charges in the constriction relative to the wildtype. In further embodiments, the MspA mutant may comprise an increased number of positive charges in the constriction relative to the wildtype. In further embodiments, the mutant MspA may comprise additional charges in the vestibule. It will be clear to the person skilled in the art, that further MspA mutants may be used, such as with essentially the same charge distributions, without deviating from the scope of the invention.

Hence, in embodiments, the method may comprise providing a nanopore protein, wherein the nanopore protein provides the nanopore. In further embodiments, the nanopore protein may be comprised by (or: "arranged in") the membrane, especially such that a molecule, such as the peptide, could pass from a cis side of the membrane via a nanopore in the nanopore protein to a trans side of the membrane. In embodiments, the membrane may comprise a lipid membrane, especially a lipid bilayer.

In general, it may be preferable to use a nanopore comprised by or formed by a nanopore protein. Nanopores provided by nanopore proteins may be more consistent in shape and performance than nanopores in synthetic membranes, as nanopores in synthetic membranes may vary in shape and/or size due to their production process.

The term "cis side" of the nanopore (or membrane) may herein especially refer to the side of the nanopore that an analyte, herein especially the peptide, more especially the peptide-oligonucleotide complex, is provided to. In particular, during the method, the analyte may be pulled from the cis side to a trans side by a force, such as a force provided by imposing a potential difference over the membrane.

In embodiments, the peptide, especially the peptide-oligonucleotide complex, may be provided to a cis side of the nanopore, and the oligonucleotide translocase may be provided to the cis side of the nanopore. In such embodiments, the oligonucleotide translocase may associate with the peptide-oligonucleotide complex prior to (at least part of) the oligonucleotide complex entering the nanopore. Thereby, the oligonucleotide translocase may act as an anchor to prevent the entire peptide-oligonucleotide complex to pass through the nanopore prior to the oligonucleotide translocase associating therewith to translocate the peptide-oligonucleotide complex through the nanopore with associated benefits regarding the translocation regularity and rate.

In embodiments, the nanopore, especially the nanopore protein, or especially the solid state nanopore (or the synthetic membrane), may provide a translocase position, especially a translocase position relative to the constriction, wherein the translocase position is suitable for hosting the oligonucleotide translocase during at least part of the method. Essentially, the oligonucleotide translocase may, during at least part of the method, sit on the translocase position as it translocates the oligonucleotide. Hence, in embodiments, the oligonucleotide translocase may be arranged at the translocase position during at least part of the oligonucleotide translocation.

In embodiments, the oligonucleotide translocase may bind the peptide-oligonucleotide complex at an anchor point. Especially, the oligonucleotide may, during the method, be bound to the oligonucleotide at the anchor point. The term "anchor point " may herein especially refer to a point of first contact between the translocase and the peptide-oligonucleotide complex, especially the oligonucleotide, as seen from the nanopore. Hence, the peptide-oligonucleotide complex may be considered free along its length between the constriction (where force may be applied) and the anchor point (where it may be associated, especially bound, to the translocase).

The distance between the anchor point and the constriction of the nanopore may at least partially determine the length of the peptide that may be read while the peptide is being translocated. In particular, as the oligonucleotide translocase translocates the peptide-oligonucleotide complex by translocating the oligonucleotide, it may at some point encounter the peptide (part) (or a linker) of the peptide-oligonucleotide complex. At that point, the oligonucleotide translocase may dissociate from the peptide-oligonucleotide complex. Hence, in embodiments wherein the oligonucleotide translocase translocates the complex by pulling it through the nanopore, the distance between the anchor point of the oligonucleotide translocase and the constriction may at least partially determine the length of the peptide that may be read while the peptide is being translocated. Hence, it may be beneficial to have a large distance d₁ between the anchor point and the constriction of the nanopore. In embodiments, a nanopore protein may be selected to provide a distance di between the anchor point and the constriction of the nanopore. In further embodiments, the distance di may be at least 3 nm, such as at least 5 nm, especially at least 7 nm, such as at least 8 nm, especially during at least part of the translocation.

As the translocase may move relative to the nanopore, the distance di may vary during the translocation of the peptide-oligonucleotide complex through the nanopore. However, in general, the translocase may essentially be configured at the translocase position. Hence, the distance d₁ may especially refer to the distance between the constriction and the anchor point as the translocase is arranged at the translocase position.

The distance di may further affect the thermal motion of the peptide-oligonucleotide complex. Specifically, the thermal motion of the oligonucleotide may be proportional to the square root of di. These thermal fluctuations may reposition the oligonucleotide in the constriction at a fast timescale, thereby increasing the number of amino acids or bases that simultaneously affect the ion current. As the number of amino acids contributing to the ion current increases, so may the difficulty in deconvoluting their contributions, which may be detrimental to peptide characterization. Hence, in further embodiments the distance di may be at most 200 nm, such as at most 100 nm, such as at most 50 nm, especially at most 40 nm, such as at most 30 nm.

A nanopore protein may generally provide translocase positions at both sides of the nanopore. These translocase positions may provide different distances d₁ between the anchor point of a translocase arranged at the translocase positions and the constriction of the nanopore. In embodiments, the peptide, especially the peptide-oligonucleotide complex, may be provided to the side of the nanopore at which the corresponding translocase position is furthest from the constriction. Hence, the side of the nanopore corresponding to the translocase position (or: "anchor point") furthest from the constriction may generally correspond to the cis side of the nanopore.

When the oligonucleotide translocase translocates the complex, encounters the peptide (part of the complex) and dissociates, the peptide-oligonucleotide complex may (start to) move through the nanopore, especially in a direction opposite of the translocation direction of the oligonucleotide translocase. As this occurs, (a second molecule of) the oligonucleotide translocase may associate with the oligonucleotide, and may repeat the translocation of the peptide through the nanopore. Thereby, the same peptide may be (at least partially) translocated through the peptide a plurality of times, which may be beneficial for characterizing the peptide. In particular, independent observations may be obtained of the same peptide through the same nanopore. The multi-translocation of the peptide through the nanopore by (different molecules of) the oligonucleotide translocase is herein referred to as "multi-loading" of the peptide-oligonucleotide complex by the oligonucleotide translocase. The probability of multi-loading may increase with an increasing concentration of the oligonucleotide translocase.

Hence, in embodiments, the oligonucleotide translocase may be provided at a concentration sufficient to provide multi-loading of the peptide-oligonucleotide complex, especially by the oligonucleotide translocase, especially such that at least 30% of oligonucleotide translocase dissociation events are followed by a re-association of (a second molecule of) the oligonucleotide translocase, such as at least 40%, especially at least 50%, such as at least 70%, especially at least 90%. In further embodiments, the oligonucleotide translocase may be provided at a concentration sufficient such that on average the peptide-oligonucleotide complex is translocated by (different molecules of) the oligonucleotide translocase at least twice, such as at least thrice, especially at least four times, such as at least five times.

Hence, in further embodiments, the oligonucleotide translocase may be provided at a concentration of at least 100 nM, especially at least 300 nM.

In further embodiments, the oligonucleotide translocase may be linked to the nanopore, especially to a nanopore protein. Thereby, a local concentration of the oligonucleotide translocase close to the nanopore may be increased.

Similarly, in further embodiments, the oligonucleotide translocase may be linked to the membrane, especially to a synthetic membrane, or especially to a lipid membrane Thereby, a local concentration of the oligonucleotide translocase close to the nanopore may be increased.

Hence, in further embodiments, the oligonucleotide translocase may be provided at a local concentration close to the nanopore of at least 100 nM, especially at least 300 nM. In further embodiments, the nanopore protein may comprise MspA or a mutant thereof. MspA is a nanopore protein from *mycobacterium smegmatis.* MspA may be suitable for high-accuracy nanopore sequencing. MspA may provide a suitable distance between a translocase position and the constriction. For example, with Hel308 as oligonucleotide translocase and arranged at the translocase position, a distance di between the anchor point and the constriction may be about 9 nm, which may correspond to the length of about 26 amino acids. Further, the structure of MspA may be relatively well understood and MspA may have been engineered through point mutations for oligonucleotide sequencing purposes. Additionally, MspA may be low-noise, may readily insert into a lipid (bilayer) membrane, and may be stable in a wide range of temperatures and pH. Hence, MspA may be particularly suitable for characterizing a peptide-oligonucleotide complex.

In embodiments, the method may comprise exposing the peptide-oligonucleotide complex to the oligonucleotide translocase, wherein the oligonucleotide translocase is configured to translocate the oligonucleotide such that at least part of the peptide translocates through the nanopore.

In embodiments, the oligonucleotide may comprise a DNA oligonucleotide, and the oligonucleotide translocase may comprises an enzyme selected from the group comprising DNA-translocases, especially a DNA-translocase selected from the group comprising a polymerase and a helicase. In further embodiments, the oligonucleotide translocase may comprise an enzyme selected from the group comprising Hel308 helicase, Phi29 polymerase, or a mutant of either. In further embodiments, the oligonucleotide translocase may comprise Hel308 or a mutant thereof, especially Hel308. Hel308 is a DNA helicase from the extremophile archaea *thermococcus gammatolerans.* Hel308 may be stable in a wide range of temperatures and pH. When Hel308 controls DNA translocation through a nanopore, the translocation may provide two observation locations per nucleotide of DNA due to the step pattern and size of Hel308. Since the length of an amino acid measured along the peptide backbone may be approximately half the length of a DNA base measured along a DNA backbone, Hel308 may provide about one observation location per amino acid. Thereby, Hel308 may be particularly suitable as an oligonucleotide translocase for a peptide-oligonucleotide complex.

In further embodiments, the oligonucleotide translocase may comprise Phi29 or a mutant thereof.

The term "mutant" (or "derivative") with regards to a first protein will be understood to refer herein to a mutant protein with one or more amino acid modifications and/or amino acid substitutions and/or amino acid deletions relative to the first protein, while retaining a similar function as the first protein. In embodiments, the mutant protein may have a sequence identity of at least an identity threshold, such as at least 30%, with respect to a sequence alignment between the sequence of the mutant with the first protein, wherein the sequence alignment has a length of at least ≥ 50% of the sequence length of the first protein, such as ≥ 60%, especially ≥ 70%, such as ≥ 80, especially ≥ 90, such as ≥ 99%, including 100%. In particular the identity threshold may be at least 30%, such as at least 40%, especially ≥ 50%, such as ≥ 60%, especially ≥ 70%, such as ≥ 75%, especially ≥ 80%, such as ≥ 85%, especially ≥ 90%, such as ≥ 93%, especially ≥ 95%, such as ≥ 97%, including 100%.

In embodiments, the method may comprise imposing a potential difference over the nanopore, especially between a cis side of the nanopore and a trans side of the nanopore. In further embodiments, the potential difference may be selected to be suitable to move (or "draw") the peptide-oligonucleotide complex into the nanopore. In further embodiments, the potential difference may be selected from the range of 10 - 400 mV.

In further embodiments, the potential difference may be varied during translocation of the peptide (by the oligonucleotide translocase). By varying the potential difference, the resulting force pulling the peptide-oligonucleotide complex towards the trans side of the nanopore may vary, and may thereby move the peptide-oligonucleotide complex relative to the constriction. Thereby, as the oligonucleotide translocase steps along the oligonucleotide in (regularly sized) steps, the peptide-oligonucleotide may also be moved back and forth with respect to the constriction in a continuous fashion, especially allowing to adjust which part of the peptide is arranged at the constriction. For example, if a particular amino acid were arranged at the constriction at any point during the method, by varying the imposed potential difference also the surroundings, such as neighboring amino acids, of this particular amino acid could be (temporarily) arranged at the constriction.

Such embodiment may be particularly beneficial when observing the current through the nanopore, as the obtained measurements for the surroundings of a measurement location may be informative with regards to adjacent measurement locations. In particular, an oligonucleotide translocase may (occasionally) move irregularly, such as take quick consecutive steps, take a larger than usual step, or take a backwards step, which may typically be difficult to detect and correct for in the resulting data. However, when the data includes measurements of the surroundings of at least part of the measurement locations, this may enable to identify occurrences of the oligonucleotide translocase moving irregularly.

Hence, in embodiments, the method may comprise varying the potential difference in a voltage range, especially varying the potential difference in the voltage range of 10 - 400 mV, especially in the voltage range of 20 - 300 mV, such as in the voltage range of 100 - 200 mV, especially between two consecutive steps of the oligonucleotide translocase along the oligonucleotide, *i.e.,* a voltage range may be measured between (each of the) consecutive steps of the helicase. In particular, essentially the entire voltage range, for example of 10 - 400 mV, may be measured between (each of) the consecutive steps of the helicase. Further, the method may comprise varying the potential difference, especially over the entire voltage range, at a frequency selected such that the period (of the voltage change) is average time between consecutive steps of the oligonucleotide translocase, especially at a frequency of at least 5 Hz, especially at least 10 Hz, such as at least 20 Hz. The frequency may especially depend on the used oligonucleotide-translocase. For example, about 200 Hz may be used with Hel308. In further embodiments, the method may comprise varying the potential difference using a (symmetrical) triangle wave, especially as described in Noakes et al., "Increasing the accuracy of nanopore DNA sequencing using a time-varying cross membrane voltage", Nature Biotechnology, volume 37, 2019, which is hereby herein incorporated by reference.

By imposing a potential difference across the nanopore, ions may flow from one side of the nanopore to the other side of the nanopore to lower their electrical potential. This phenomenon may be used in nanopore oligonucleotide sequencing to draw an oligonucleotide, which may generally comprise negative charges throughout the polymer, into the nanopore. Peptides, however, may vary in charge distribution along the polymer, and may typically comprise a mixture of positive and negative charges, and may vary in the extent to which they are drawn into the nanopore by the imposing of a potential difference over the nanopore.

In embodiments, however, the method may comprise drawing the peptide-oligonucleotide complex into the nanopore peptide-first. Hence, the peptide-oligonucleotide complex may have a peptide side and an oligonucleotide side, and the method may comprise leading the peptide side of the complex into the nanopore (from the cis side). Such embodiments may be particularly suitable when the oligonucleotide translocase is provided at the cis side of the nanopore, and may associate with the oligonucleotide prior to the peptide-oligonucleotide complex entering the nanopore.

In further embodiments, the method may comprise drawing the peptide-oligonucleotide complex into the nanopore oligonucleotide-first. Hence, the peptide-oligonucleotide complex may have a peptide side and an oligonucleotide side, and the method may comprise leading the oligonucleotide side of the complex into the nanopore, especially from the trans side, or especially from the cis side, and especially wherein an oligonucleotide translocase is arranged at the other side of the nanopore, such as at the cis side of the nanopore.

In further embodiments, the peptide-oligonucleotide complex may comprise a negatively charged element arranged at the peptide side. The negatively charged element may be configured to move into the nanopore when a (suitable) potential difference is imposed across the nanopore. In further embodiments, the negatively charged element may especially comprise at least 1 negative charge, especially at least 3 negative charges, such as at least 5 negative charges, especially at least 10 negative charges. In further embodiments, the negatively charged element may comprise one or more of a second oligonucleotide, a charged peptide, and a charged non-peptide chemical species, especially one or more of a second oligonucleotide and a charged peptide. In general, the second oligonucleotide may be different from the oligonucleotide, *i.e.,* the second oligonucleotide and the first oligonucleotide may have different nucleotide sequences. In embodiments, however, the second oligonucleotide and the oligonucleotide may also have the same nucleotide sequence. Regardless, the second oligonucleotide and the oligonucleotide may be distinct oligonucleotides, *i.e.,* there are two separate oligonucleotides linked to the ends of the peptide rather than a single oligonucleotide that connects to both ends of the peptide.

Hence, in embodiments, the peptide-oligonucleotide complex may be (essentially) linear.

The negatively charged element may be attached to the peptide via a linker.

Hence, in embodiments, the method may comprise attaching a negatively charged element to the peptide (via a linker), especially at an opposite end of the peptide with respect to the oligonucleotide.

Hence, in embodiments, the peptide may have a first peptide end and a second peptide end, wherein the first peptide end is linked to the oligonucleotide, and wherein the second peptide end is linked to a negatively charged element, especially wherein the negatively charged element is selected from the group comprising a second oligonucleotide, a charged peptide, and a charged non-peptide chemical species, especially from the group comprising a second oligonucleotide and a charged peptide.

Hence, in embodiments, the peptipde-oligonucleotide complex may comprise a negatively charged element at a second peptide end of the peptide, and the method may comprise drawing the peptide-oligonucleotide complex in from the negatively charged element (i.e., the negatively charged element enters the nanopore first). Hence, the peptide may follow the negatively charged element into the nanopore. Further, an oligonucleotide translocase may associate with the oligonucleotide of the peptide-oligonucleotide complex, especially wherein the oligonucleotide translocase controls (at least part of) the translocation of the peptide through the nanopore. In further embodiments, the negatively charged element may comprise a second oligonucleotide.

In further embodiments, the method may comprise sensing a translocation related signal, especially an electrical current signal, as the peptide-oligonucleotide complex translocates through the nanopore. The translocation related signal may relate to one or more of the negatively charged element, the peptide, and the oligonucleotide, especially at least to the peptide. In particular, the translocation related signal may facilitate characterizing the peptide, i.e., the method may further comprise characterizing the peptide based on the translocation related signal. The peptide part of the peptide-oligonucleotide complex may provide a distinct translocation related signal relative to the oligonucleotide and the negatively charged element, such as a second oligonucleotide. Hence, based on the translocation-related signal, the end of the peptide may be identified, i.e., the method may comprise identifying the first end and/or the second end of the peptide based on the translocation-related signal, which may further be beneficial for peptide characterization as, for instance, this information may be combined with information pertaining to the method of protein fragmentation.

In embodiments, the oligonucleotide may be (at least partially) hybridized with (or "bound to") a complementary oligonucleotide, especially during at least part of the method, more especially prior to the oligonucleotide entering the nanopore, thereby providing a double helix. In particular, the oligonucleotide translocase may be (selected to be) inefficient in unwinding the double helix, especially the oligonucleotide translocase may especially (be selected to) essentially not unwind the double helix. In particular, the oligonucleotide translocase may be associated with the oligonucleotide, but may essentially not move relative to the oligonucleotide due to the complementary oligonucleotide.

Hence, in embodiments, the method may comprise providing a complementary oligonucleotide, wherein the complementary oligonucleotide is at least partially complementary to the oligonucleotide.

As the peptide-oligonucleotide complex is drawn into the nanopore, for example due to the imposed potential difference, the force may shear off the complementary oligonucleotide, especially as the double helix may be too large to enter the nanopore, which may result in dissociation of the double helix. As the complementary oligonucleotide (fully) dissociates from the oligonucleotide, the oligonucleotide translocase may start to translocate the oligonucleotide, especially thereby pulling the peptide-oligonucleotide complex back through the nanopore. Thereby, the complementary oligonucleotide, especially the double helix, may function as a blocker, and may (be configured to) facilitate reading the peptide from an end of the peptide.

In further embodiments, the complementary oligonucleotide may comprise a plurality of complementary oligonucleotides, wherein each of the plurality of complementary oligonucleotides is at least partially complementary to the oligonucleotide. In particular, two of the complementary oligonucleotides may be configured to be complementary to two sections of the oligonucleotide, wherein the two sections are separated by a single base. Hence, when the two of the complementary oligonucleotides are bound to the oligonucleotide, there may be an unpaired base (also a "nick") in the oligonucleotides, especially in a double helix comprising the oligonucleotides, between the two sections of the oligonucleotide bound to the two of the complementary oligonucleotides. Such embodiments may be particularly beneficial with oligonucleotide translocases that bind a double-stranded oligonucleotide but essentially only start actively translocating the oligonucleotide once they encounter a nick. For example, in further embodiments, the oligonucleotide translocase may comprise the Phi29 DNA polymerase. The Phi29 DNA polymerase may bind the oligonucleotide and act as an anchor and brake as the oligonucleotide translocates through the nanopore due to an imposed potential difference. Once the Phi29 DNA polymerase encounters the nick, as one of the plurality of complementary oligonucleotides dissociates from the oligonucleotide, the Phi29 DNA polymerase may start actively translocating the oligonucleotide, especially by generating the complementary strand of the oligonucleotide, thereby pulling it back through the nanopore, especially translocating it from the trans side to the cis side of the nanopore, enabling a reverse translocation (and analysis) of the oligonucleotide.

Hence, in further embodiments, the plurality of complementary oligonucleotides may be configured to provide a nick on a complementary strand of the oligonucleotide, especially a plurality of nicks.

Further, the complementary oligonucleotide may be associated, especially linked, to a tag configured to associate with the membrane, especially with a lipid membrane. For example, the tag may comprise a cholesterol tag, which may associate with a lipid membrane. Thereby, the complementary oligonucleotide, as well as bound oligonucleotide and therewith attached oligonucleotide translocase, may have an increased local concentration near the membrane, which may increase the rate at which the peptide-oligonucleotide complex enters the nanopore. In particular, in embodiments wherein the membrane comprises a lipid membrane, the membrane may be considered as a two dimensional liquid, and everything in it (including the nanopore and the associated oligonucleotides) may diffuse around freely. Association with the membrane may, however, reduce the space of the diffusion from a 3D volume to a 2D slice through that volume, which may drastically increase the likelihood of a peptide-oligonucleotide complex encountering the nanopore, and may correspondingly increase the speed of the method, especially at lower concentrations of peptide-oligonucleotide complexes.

Hence, in embodiments, the nanopore may be comprised by a membrane, wherein the method comprises providing a complementary oligonucleotide at least partially complementary to the oligonucleotide. The complementary oligonucleotide may be associated, especially linked, to a tag. In further embodiments, the tag may (be configured to) associate with the (lipid) membrane. The phrase "the tag associates with the membrane" and similar phrases may herein refer to the tag functionally coupling with the membrane, *i.e.,* the tag may insert into the membrane. In particular, the tag may insert into the membrane, and may move around in the membrane. Thereby, the oligonucleotide complex hybridized with the complementary oligonucleotide may have a locally increased concentration close to the membrane. Especially, the diffusion of the oligonucleotide complex hybridized with the complementary oligonucleotide may essentially be 2-dimensional along the membrane rather than 3-dimensional as in free solution, which may increase the rate at which the oligonucleotide complex encounters the nanopore.

In further embodiments, the tag may comprise cholesterol.

Some oligonucleotide translocases may have a nucleolytic function, *i.e.,* they may cut oligonucleotides, which may be undesirable for the method. Hence, in embodiments, the oligonucleotide translocase may be selected from the group comprising non-nucleolytic enzymes.

In embodiments, the method may comprise a complex-formation step. The complex-formation step may comprise (chemically) linking the peptide and the oligonucleotide thereby providing the peptide-oligonucleotide complex. Methods to link a peptide and oligonucleotide will be known to the person skilled in the art. For example, in embodiments, the complex formation step may comprise linking the peptide and the oligonucleotide via a procedure selected from the group comprising 5' copper free click chemistry and maleimide-thiol linkage.

Hence, the peptide-oligonucleotide complex may further comprise a linker arranged between the peptide and the oligonucleotide, *i.e.,* the peptide and the oligonucleotide may be linked via a linker, especially a linker obtained using 5' copper free click chemistry and/or maleimide-thiol linkage.

In embodiments, the nanopore may be arranged between a first electrolyte and a second electrolyte. The first electrolyte may especially be arranged at the cis side (of the nanopore). The second electrolyte may especially be arranged at the trans side (of the nanopore). The first electrolyte and the second electrolyte may especially be in fluid communication via the nanopore. In further embodiments, the first electrolyte and the second electrolyte may especially be (essentially) the same electrolyte. In further embodiments, the first electrolyte and the second electrolyte may comprise a salt concentration independently selected from the range of 1 mM - 10 M, especially from the range of 200 mM to 3 M, such as from the range of 50 mM to 1 M. In further embodiments, the first electrolyte and the second electrolyte may have a pH independently selected from the range of 3 - 9, especially from the range of 5 - 8.

In embodiments, the first electrolyte and the second electrolyte may be selected to provide denaturing conditions for the peptide, especially such that the peptide is essentially in a primary structure. In further embodiments, the first electrolyte and/or the second electrolyte may comprise a denaturant.

Further, the first electrolyte and the second electrolyte may be selected to be suitable for the oligonucleotide translocase. As will be clear to one skilled in the art, different translocases may have different requirements with regards to, for example, salt concentration, pH, temperature, and/or cofactors. For example, at more extreme conditions the thermophilic helicase Hel308 may operate better than the bacteriophage polymerase Phi29. Changing aforementioned parameters may change the processivity and walking kinetics of the translocase, and it may even be the case that different choices give different advantages. For example, providing saturating NTP concentration, especially ATP concentration, to Hel308 increases the processivity and the rate at which reads are obtained (relative to a low NTP concentration), but decreases the time spent in observing each state. This means that high-NTP conditions might be good for rapid screening to detect a known molecule, whereas low-NTP conditions may be good for de novo sequencing, for example.

For example, for Hel308 as oligonucleotide translocase, a saturating NTP, especially ATP, concentration may be ≥ 1 mM NTP, especially ATP. Similarly, for Hel308, a low NTP concentration, facilitating relatively long observation times, may be ≤ 100 µM, such as ≤ 50 µM.

Hence, in embodiments, the first electrolyte may comprise an NTP concentration selected from a range of 10 µM - 2 mM. In further embodiments, the NTP concentration may especially be an ATP concentration.

As there may be a trade-off in measurement speed and measurement accuracy (due to observation times), the oligonucleotide translocase, as well as the NTP concentration, may be tuned to satisfy the specific requirements for a particular embodiment of the method. In general, however, the oligonucleotide translocase may be configured to process the oligonucleotide at a rate selected from the range of 0.5-1000 nucleotides/second. This range may generally provide both a suitable measurement speed and measurement accuracy.

In embodiments, the first electrolyte and the second electrolyte may be selected to be suitable for the nanopore protein.

In embodiments, the method may comprise a digestion stage. The digestion stage may comprise fragmenting the protein to provide the peptide, especially a plurality of peptides. In further embodiments, the digestion stage may comprise exposing the protein to one or more of an enzymatic digestion and a chemical digestion, such as by exposing the protein to a protease or by exposing the protein to a (weak) acid. It will be clear to the person skilled in the art that a protein may be fragmented differently depending on, for example, the choice of protease or chemical digestant, especially such that peptides with a particular end group may be obtained for linking of the peptide to an oligonucleotide.

For example, a terminal cysteine group may facilitate linking of the peptide, such as via maleimide-thiol linkage.

In a second aspect, the invention may provide an analysis method for analyzing a peptide. The analysis method may especially comprise the method for translocating a peptide through the nanopore. In embodiments, the analysis method may comprise sensing a translocation-related signal during the translocation.

In further embodiments, the analysis method may comprise measuring the translocation of the peptide through the nanopore to provide a translocation-related signal.

As the method for translocating a peptide through the nanopore may provide a steady (or "regular") peptide translocation through the nanopore (with regards to step-size and/or step-rate), it may facilitate analyzing the peptide as measurements corresponding to different parts of the peptide, especially different amino acids of the peptide, being arranged in the nanopore, especially near the constriction, may be temporally resolved.

In embodiments, the analysis method may comprise imposing a potential difference over the nanopore, especially wherein the analysis method further comprises measuring an electrical current through the nanopore and providing an electrical current signal. In particular, the translocation-related signal may comprise the electrical current signal.

In embodiments, the analysis method may further comprise an optical read-out of the translocation of the peptide through the nanopore, especially wherein the optical read-out comprises a FRET measurement, and providing an optical read-out related signal. In particular, the translocation-related signal may comprise the optical read-out related signal.

In embodiments, the analysis method may further comprise a characterization stage, wherein the characterization stage comprises characterizing the peptide based on (processing of) the translocation-related signal. Hence, the translocation-related signal may be characteristic of the peptide. In particular, the translocation-related signal may comprise a "peptide fingerprint" suitable to identify the peptide.

Hence, in embodiments, the analysis method may comprise peptide fingerprinting.

In embodiments, the characterization stage may comprise identifying one or more amino acids. In further embodiments, the characterization stage may comprise one or more post-translational modifications. In particular, the analysis method may be suitable to identify distributions of post-translational modifications in a peptide population, especially when there is a relation between the presence and/or absence of two or more post-translational modifications, such as for two or more post-translational modifications that are (essentially) mutually exclusive or co-occurring. Observing mutually exclusive and/or co-occurring post-translational modifications may be difficult with prior art techniques, such as mass spectrometry, especially due to the fragmentation following ionization.

In embodiments, the characterization stage may comprise determining a protein characteristic selected from the group comprising presence, location, and/or identity of post-translational modifications; presence, location and/or identity of single-amino-acid variants; peptide identity; protein identity; peptide sequence; splice variants; relative or absolute peptide (or protein) concentration; and secondary structure formation.

In embodiments, the characterization stage may comprise an identification stage. The identification stage may comprise identifying the peptide based on the translocation-related signal, especially by comparing the translocation-related signal to (pre-measured) reference translocation-related signals of known peptides from a database, or especially by comparing the translocation-related signal to predicted translocation-related signals of known peptides from a database. In further embodiments, the identification stage may comprise identifying a protein, especially based on the translocation-related signal, or especially based on the identified peptide, more especially based on a plurality of identified peptides. In particular, the peptide may be derived from the protein, e.g., via enzymatic or chemical protein digestion, and the protein may be identified based on the translocation-related signal of the peptide, especially based on the identified peptide.

In further embodiments, the identification stage may comprise (at least partially) sequencing the peptide, i.e., the identification stage may comprise identifying one or more amino acids in the peptide based on the translocation-related signal. In further embodiments, the identification stage may comprise *de novo* sequencing based on the translocation-related signal. In further embodiments, the identification stage may comprise reference sequencing based on the translocation-related signal. The identification stage may especially comprise computationally identifying one or more amino acids in the peptide based on the translocation-related signal, especially using an algorithm.

For example, the identification stage may comprise: 1. Preprocessing of data: filtering the translocation-related signal, especially a raw ion current stream, identifying and classifying blockages, finding events corresponding to peptide reads, and scoring the quality of those events using heuristics or machine learning classifiers (such as a perceptron or support vector machine). In the case of a variable-voltage read: calculating and removing the capacitive ion current. 2. Segmenting data: Using either a hidden Markov model (in the case of reference sequencing) or a greedy maximum likelihood binary segmentation algorithm to segment data. 3. Sequencing: Using a custom hidden Markov model or a recurrent neural network to either perform *de novo* or reference sequencing. 4. Identification: Using genomic methods, such as customized BLAST searches through (nanopore) databases to identify the reads. 5. Advanced analysis: Using special custom algorithms based on machine learning, hidden Markov models, dynamic programming, and optimization techniques to train the models used in other steps, build consensuses of multiple reads to improve accuracy, assemble short peptide reads into full protein reads, and assess the statistical accuracy and robustness of the methods.

It will be clear to the person skilled in the art that many (combinations of) (bioinformatic) methods may be used for identifying one or more amino acids in the peptide based on the translocation-related signal without deviating from the scope of the invention as set out herein.

As will be clear to the person skilled in the art, the analysis method may essentially be performed on-site with a small device. Hence, it may be preferable to use proteins that are stable, especially with regards to (extreme) temperatures such as at the poles or in the desert, such as Hel308 and MspA.

The analysis method is essentially a single-molecule technology, as a single molecule may pass through the nanopore at a time. Thereby, in principle, every single individual molecule that passes through the nanopore can be analyzed. Hence, as long as there is a high enough concentration of analyte to expect at least one interaction with the nanopore in the entire lifetime of the experiment, it can be measured. In particular, with the (membrane-associating) tag, and long-lived and/or high-throughput measurement devices (such as parallelized nanopore arrays), the sensitivity of the analysis method may be further improved. Hence, in embodiments the analysis method may facilitate characterizing peptides at a concentration in the attomolar range. In further embodiments, the analysis method may facilitate characterizing peptides at a concentration in the femtomolar range. the analysis method may facilitate characterizing peptides at a concentration in the picomolar range.

In a further aspect, the invention may provide the use of an oligonucleotide translocase to translocate a peptide-oligonucleotide complex, especially through a nanopore.

In embodiments, the oligonucleotide translocase may comprise Hel308, Phi29 or a mutant of either. Especially, the oligonucleotide translocase may comprise Hel308 or a mutant thereof.

In a further aspect, the invention may provide the use of a nanopore for characterizing, especially sequencing, a peptide-oligonucleotide complex. In embodiments, the use may be for characterizing, especially sequencing, a peptide, wherein the peptide is comprised by a peptide-oligonucleotide complex.

In embodiments, the nanopore may be provided by a nanopore protein, wherein the nanopore protein comprises MspA or a mutant thereof.

In a further aspect, the invention may provide a kit of parts comprising one or more of an oligonucleotide translocase, a buffer comprising ATP, an oligonucleotide, a linker suitable to link the oligonucleotide to a peptide, and an enzyme for fragmenting peptides.

The kit of parts may provide the benefit that the method of the invention may be (conveniently) executed with existing nanopore sequencing infrastructure.

In embodiments, the kit of parts may further comprise a data storage element comprising analysis software.

In embodiments, the kit of parts may comprise the oligonucleotide translocase. In further embodiments, the kit of parts may comprise the buffer comprising NTP, especially ATP. In further embodiments, the kit of parts may comprise the oligonucleotide. In further embodiments, the kit of parts may comprise the linker suitable to link the oligonucleotide to a peptide. In further embodiments, the kit of parts may comprise the enzyme for fragmenting peptides.

In specific embodiments, the kit of parts may comprise an oligonucleotide translocase, a buffer comprising NTP, especially ATP, an oligonucleotide, and a linker suitable to link the oligonucleotide to a peptide.

In a further aspect, the invention may provide a use of a nanopore for fingerprinting and/or sequencing a protein, especially fingerprinting a protein, or especially sequencing a protein.

In a further aspect, the invention may provide a use of a nanopore for characterizing posttranslational modifications on a protein.

The embodiments described herein are not limited to a single aspect of the invention. For example, an embodiment describing a nanopore protein in relation to the method for translocating a peptide may further apply to the use of a nanopore for characterizing, especially sequencing, a peptide-oligonucleotide complex. Similarly, an embodiment describing an oligonucleotide translocase in relation to the method for translocating a peptide may further apply to the kit of parts.

The invention may, for example, be applied for the characterization of proteins and peptides, especially in relation to identifying or sequencing short peptides with relevance to immunology, cancer immunotherapy and other personalized medicines, pharmacology, or fundamental biology, identifying or sequencing biologically or medically relevant full-length proteins through shotgun sequencing, identifying or sequencing low-concentration proteins that are difficult to detect, especially essentially undetectable, with prior art methods such as by mass spectrometry, identifying protein or peptide markers of a particular pathology or organism, quality control in pharmaceuticals or industrial production of biological materials, and quality control in agriculture and food production and monitoring.

### BRIEF DESCRIPTION OF THE DRAWINGS

Embodiments of the invention will now be described, by way of example only, with reference to the accompanying schematic drawings in which corresponding reference symbols indicate corresponding parts, and in which: Fig. 1 schematically depicts an embodiment of the method. Fig. 2 schematically depicts another embodiment of the method. Fig. 3 schematically depicts experimental data obtained using an embodiment of the analysis method. Fig. 4 schematically depicts another embodiment of the method. Fig. 5 schematically depicts experimental data obtained using an embodiment of the analysis method. Fig. 6 schematically depicts experimental data obtained using an embodiment of the analysis method. The schematic drawings are not necessarily on scale.

### DETAILED DESCRIPTION OF THE EMBODIMENTS

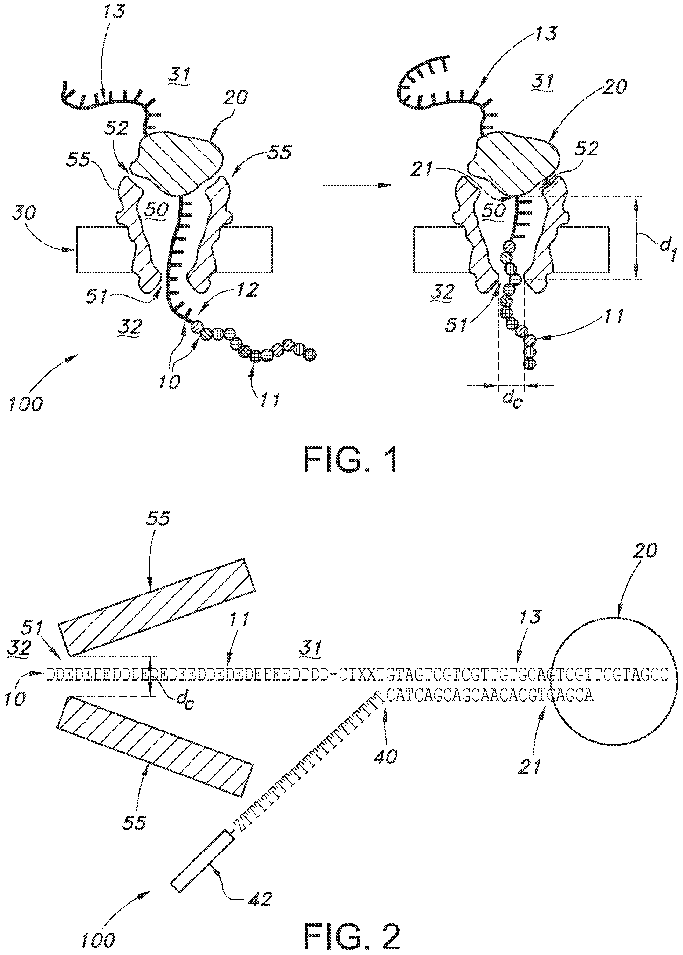

Fig. 1 schematically depicts an embodiment of the method 100 for translocating a peptide 11 through a nanopore 50 in the presence of an oligonucleotide translocase 20. In the depicted embodiment, the peptide 11 is comprised by a peptide-oligonucleotide complex 10, wherein the peptide 11 is linked to an oligonucleotide 13. The oligonucleotide translocase 20 affects the translocating of the peptide-oligonucleotide complex 10 through the nanopore 50, especially the oligonucleotide translocase 20 moves the oligonucleotide 13 relative to the oligonucleotide translocase 20. In particular, the oligonucleotide translocase 20 pulls the oligonucleotide 13, and thereby the peptide-oligonucleotide complex 10, through the nanopore 50 towards the oligonucleotide translocase 20, especially towards the cis side 31 of the nanopore (or membrane), thereby translocating at least part of the peptide-oligonucleotide complex 10 through the nanopore 50.

Specifically, figure 1 may depict an embodiment of the method wherein the peptide 11 is in the constriction 51 of the nanopore 50 while the oligonucleotide translocase 20 is walking along the oligonucleotide 13. The (sensitive) constriction is distanced from the anchoring oligonucleotide translocase by a distance di. This may enable a scheme in which a peptide 11 (chain) is being moved through the constriction 51 while an oligonucleotide translocase 20 walks along a conjugated oligonucleotide 13 (a). First, on the left side of the image, the conjugated oligonucleotide 13 is arranged at the constriction 51, especially wherein the oligonucleotide 13 is read at the constriction 51. Next, on the right side of the image, as the oligonucleotide translocase 20 walks towards the peptide 11 of the peptide-oligonucleotide complex 10, the peptide 11 is translocated through from the trans side 32 of the nanopore 50 to the cis side 31 of the nanopore, especially via the constriction 51.

In the depicted embodiment, the oligonucleotide 13 comprises a DNA oligonucleotide, and the oligonucleotide translocase 20 comprises an enzyme selected from the group comprising a polymerase and a helicase.

In the depicted embodiment, the nanopore 50 comprises a constriction 51 and an translocase position 52. The constriction 51 may have a circular equivalent diameter d_{c} selected from the range of 0.5 - 3 nm. The oligonucleotide translocase 20 may be arranged at the translocase position 52 during at least part of the oligonucleotide translocation.

The position of first contact between the oligonucleotide translocase 20 and the oligonucleotide 13, as seen from the nanopore, may herein be referred to as the anchor point 21. In embodiments, a distance di between the constriction 51 and the anchor point 21 may be at least 3 nm. In particular, the distance d₁ may refer to the distance between the constriction 51 and the anchor point 21 as the oligonucleotide translocase 20 is arranged at the translocase position 52.

In the depicted embodiment, the nanopore 50 is comprised by and formed by a nanopore protein 55. In further embodiments, the nanopore protein may comprise MspA. MspA may provide a distance di of about 9 nm.

In the depicted embodiment, the nanopore 50, especially the nanopore protein 55, is comprised by a membrane 30, especially a lipid membrane.

Fig. 1 further schematically depicts the use of an oligonucleotide translocase 20 to translocate a peptide-oligonucleotide complex 10, especially through a nanopore 50. In further embodiments, the oligonucleotide translocase 20 may comprise Hel308 or a mutant thereof.

Fig. 1 further schematically depicts the use of a nanopore 50 for characterizing a peptide-oligonucleotide complex 10. In particular, the nanopore 50 is provided by a nanopore protein 55. In further embodiments, the nanopore protein 55 comprises MspA or a mutant thereof.

Fig. 2 schematically depicts another embodiment of the method 100. In the depicted embodiment, the method 100 comprises providing a complementary oligonucleotide 40 at least partially complementary to the oligonucleotide 13. Here, the complimentary oligonucleotide 40 comprises a complementary region depicted associated with the oligonucleotide 13 thereby forming a double-stranded helix, as well as a non-complimentary region, especially a poly-T region, not associated with the oligonucleotide 13. In the depicted embodiment, the complementary oligonucleotide 40, especially the non-complimentary region, is linked to a tag 42, especially via a linker indicated with the "-Z", and especially wherein the tag 42 associates with the membrane 30 (not depicted). Thereby, the local concentration of the complementary oligonucleotide 40, and thereby of the peptide-oligonucleotide complex, can be increased close to the membrane 30, which may increase the rate at which the peptide-oligonucleotide complex 10 enters the nanopore 50.

In the depicted embodiment, the peptide 11 is provided to a cis side 31 of the nanopore 50 and the oligonucleotide translocase 20 is provided to the cis side 31 of the nanopore 50. Thereby, the oligonucleotide translocase can associate with the oligonucleotide, especially the peptide-oligonucleotide complex, prior to the peptide entering the nanopore.

In embodiments, the oligonucleotide translocase may be (selected to be) inefficient at processing, especially translocating, a double-stranded helix. As the peptide-oligonucleotide complex 10 enters the nanopore 50, the complementary oligonucleotide 40 may be sheared off, thereby exposing a single-stranded oligonucleotide 13 to the oligonucleotide translocase 20, resulting in (more efficient) processing of the oligonucleotide 13 by the oligonucleotide translocase 20.

Hence, the complementary oligonucleotide 40 may provide both an increased local concentration, and may delay the translocating of the oligonucleotide by the oligonucleotide translocase until the peptide-oligonucleotide complex 10 has entered the nanopore 50.

In further embodiments, the method 100 may comprise imposing a potential difference over the nanopore 50. In particular, by imposing the potential difference, the peptide-oligonucleotide complex 10 may be drawn into the nanopore 50. In further embodiments, the potential difference may be selected from the range of 10- 400 mV, especially from the range of 100 - 200 mV, and the method 100 may comprise varying the potential difference between two consecutive steps of the oligonucleotide translocase 20.

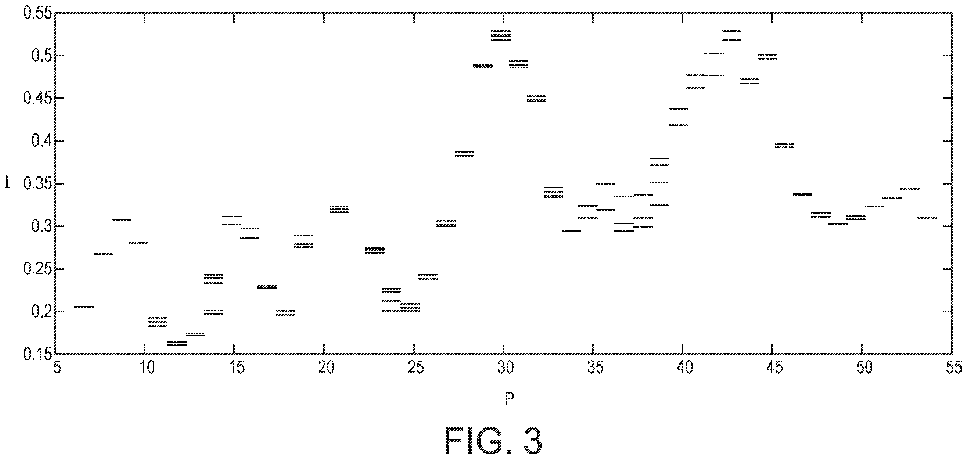

Fig. 3 schematically depicts experimental observations of the analysis method for analyzing a peptide 11. In particular, Fig. 3 depicts the translocation-related signal, especially the electrical current signal. The analysis method comprises the method 100.

In particular, the depicted results were obtained using an embodiment of the method 100 wherein a peptide-oligonucleotide complex, consisting of an oligonucleotide 13 linked at the 5' via copper free click chemistry to the C-terminus of a negatively charged peptide 11. The oligonucleotide-translocase may bind to a loading site of the oligonucleotide, and the peptide 11 may feed into the nanopore 50. The peptide-oligonucleotide complex 10, especially the oligonucleotide 13, may be bound to a complementary oligonucleotide 40, which may serve two purposes. Firstly, the oligonucleotide translocase, here especially Hel308, may not efficiently initiate unwinding at single-stranded to double-stranded junctions, so the complementary oligonucleotide may function as a blocker, preventing unwinding of the double-stranded helix. Secondly, a tag 40, here especially a cholesterol tag, at the 3' end of the complementary oligonucleotide 40 may associate with a lipid membrane 30, increasing the local concentration of the peptide-oligonucleotide complex 10 and improving the rate of capture by the nanopore 50. When the peptide-oligonucleotide complex 10 is pulled into the nanopore 50, the force may shear off the complementary oligonucleotide 40, leaving only the peptide-oligonucleotide complex 10 and the oligonucleotide translocase 20 at the nanopore 50, and enabling the oligonucleotide translocase 20 to begin walking 3' to 5', pulling the peptide-oligonucleotide complex up half a nucleotide at a time. The analysis method further comprises measuring the translocation of the peptide 11 through the nanopore 50 to provide a translocation-related signal. In particular, for the depicted embodiment, the analysis method comprised imposing a potential difference over the nanopore 50, and measuring an electrical current through the nanopore 50 and providing an electrical current signal, wherein the translocation-related signal comprises the electrical current signal.

### Examples

Proteins - the experimental procedures were performed using the M2-NNN-MspA mutant (see above) and with the Hel308 helicase enzyme from *Thermococcus gammatolerans* EJ3 (NCBI accession number WP_015858487.1).

Peptide-oligonucleotide complex - the peptide-oligonucleotide complex comprised a peptide linked to an oligonucleotide via either copper-free click chemistry or maleimide-thiol linkage (as specified). The peptide-oligonucleotide complex was associated with a complementary oligonucleotide linked to a cholesterol tag. The cholesterol tag at the 5' end of the complementary oligonucleotide may anchor the peptide-oligonucleotide complex into a lipid (bilayer) membrane, thereby increasing the local concentration near the pore and increasing the capture rate.

Nanopore experiments - experiments were performed with a device made from Teflon that contains two ~50 µL chambers (cis and trans). The two chambers are connected by a Teflon heat-shrink "u-tube", ~30 µL in volume. The cis side of the u-tube narrows into a horizontal ~20 µm aperture. Both chambers and the u-tube were filled with the operating buffers. The cis chamber was connected to ground via an Ag/AgCl electrode, while the trans-side Ag/AgCl electrode was connected to an integrating patch clamp amplifier that also supplied the positive driving voltage. A lipid bilayer was formed across the aperture using 1,2-diphytanoyl-sn-glycero-3-phosphocholine (DPhPC). Following bilayer formation, M2-NNN-MspA was added to the cis chamber to a final concentration of ~2.5 ng/mL. A single pore insertion into the bilayer was recognized by a characteristic increase in the conductance. Upon single pore insertion, the cis chamber buffer was perfused out and replaced with MspA-free buffer to prevent the insertion of additional pores. The Hel308 motor enzyme was added to the cis chamber to a final concentration of ~1 µM, and the peptide-oligonucleotide complex was added to a final concentration of ~500 pM. Hel308 loads onto the overhanging 3' end of the oligonucleotide 13 of the peptide-oligonucleotide complex 10 at the single-stranded/double-stranded junction. The peptide 11 of the peptide-oligonucleotide complex 10 is captured by the nanopore 50, and the complementary oligonucleotide 40 is sheared off as the template strand is pulled through the nanopore 50. Hel308 is too large to fit through MspA, and arrests the peptide-oligonucleotide 10 translocation once the complementary oligonucleotide has been sheared off. Now unblocked, Hel308 proceeds as a translocase from 3' to 5' along the peptide-oligonucleotide complex, incrementally pulling the oligonucleotide out of the nanopore 50 towards the cis side 31.

Operating buffers - the experiments were conducted using an identical first electrolyte and second electrolyte, wherein both the first electrolyte and second electrolyte comprised 400 mM KCl with 10 mM HEPES at pH 8.00 ± 0.05, 10 mM MgCl2, and 1000 µM ATP. The ATP-containing buffer was re-perfused at the cis side approximately once per hour to prevent depletion of ATP and accumulation of ADP. Experiments were performed at room temperature, -21 °C.

Data acquisition and Analysis - Experiments were controlled and data were acquired with custom acquisition software written in Lab View (National Instruments, version 2019) at a sampling rate of 50 kHz. The ionic current signal was low pass filtered at 10 kHz using the Bessel filter onboard the patch clamp amplifier. Ionic current traces were analyzed using custom programs written in Matlab (Mathworks, version 2019a). Reads were filtered using a custom compression filter to eliminate transient fluctuations in ionic current unrelated to translocating DNA sequence. Enzyme-controlled DNA translocation events were detected with a thresholding algorithm. The open pore ionic current value was determined for the data, and an event start was identified whenever the ionic current drops below 75% of the open pore value. The event end was identified when the ionic current returns to greater than 94% of the open pore value. Events with duration shorter than 1s, or with an average ionic current less than 10% or greater than 70% of the open pore value were discarded. Small variations in temperature, salt concentration, and electrode potentials from day-to-day, pore-to-pore, and read-to-read may cause changes in both the overall magnitude of the observed ion currents (an "offset") as well as the relative magnitudes of adjacent states (a "scale"). Each read was calibrated to the ensemble average prior to analysis using a scale and an offset calculated specifically for that read.

The examples specified below were performed according to the experimental procedures outlined hereabove unless specified otherwise.

### Example 1:

The measured peptide-oligonucleotide complex 10 is the peptide-oligonucleotide complex 10 depicted in Fig. 2. Specifically, the peptide-oligonucleotide complex 10 comprises DDEDEEEDDDEDEDEEDDEDEDEEEEDDDD-----CTXXTGTAGTCGTCGTTGTGCAG TCGTTCGTAGCC, wherein the oligonucleotide 13 comprises CTXXTGTAGTCGTCGTTGTGCAGTCGTTCGTAGCC, wherein the peptide 11 comprises DDEDEEEDDDEDEDEEDDEDEDEEEEDDDD, and wherein ----- is the linker 12, and wherein the linker is provided via click chemistry. Specifically, the linker is a connection between DBCO-C5 and Orn(N3), wherein DBCO-C5 refers to dibenzocyclooctyne connected by a 5-carbon spacer to the 5' phosphate of the DNA, and wherein Orn(N3) refers to 2-amino-5-azido-n-pentanoic acid, on the C-terminal end of the protein. The oligonucleotide 13 comprises two abasic sites (marked "X") shortly before the linker 12, consisting only of the DNA backbone. These abasic sites were included because of the recognizable ion current signal they cause, much higher than when only DNA bases are in the constriction 51. This may facilitate identification within each read of the end of the oligonucleotide 11 and the beginning of the linker 12 and peptide 11. Hence, in embodiments, the oligonucleotide may comprise one or more abasic sites, especially 1-3 abasic sites, arranged near the linker, especially at least partially arranged within 10 bases from the linker, especially at least partially arranged within 5 bases from the linker.

Fig. 3 depicts the measured electrical current signal of four independent reads aligned to one another, wherein I indicates the ionic current as a fraction of the open state of the nanopore (without the peptide-oligonucleotide complex 10 at the constriction 51), and P indicates the position of the peptide-oligonucleotide complex expressed in steps of the oligonucleotide translocase. In particular, P=5 - P=32 roughly corresponds to the oligonucleotide being arranged at the constriction 51, wherein P=26 - P=32 roughly corresponds to the abasic sites being arranged at the constriction 51. P=33 - P=37 roughly corresponds to the linker being arranged at the constriction. P=38 - P55 roughly corresponds to the peptide being arranged at the constriction 51, wherein P=38 - P=46 roughly corresponds to the first four amino acids (DDDD) being arranged at the constriction and P=47 - P=55 roughly corresponds to the next four amino acids (EEEE) being arranged at the constriction 51.

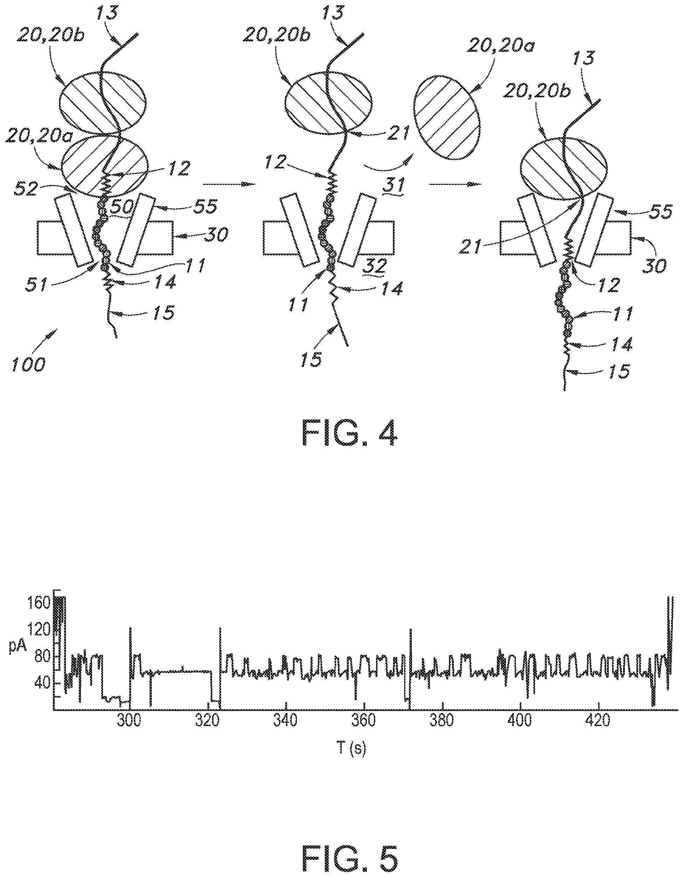

Fig. 4 schematically depicts an embodiment of the method (100) with multi-loading. Left: Multiple different oligonucleotide translocases 20,20a,20b may bind to the peptide-oligonucleotide complex 10, especially at high concentrations of the oligonucleotide translocase 20. Center: The frontmost oligonucleotide translocase 20,20a can't walk along the linker 12 or peptide 11, and dissociates from the peptide-oligonucleotide complex 10, especially when a nucleotide binding site of the oligonucleotide translocase 20 encounters the linker 12. Right: The force induced by the potential difference pulls the peptide-oligonucleotide complex 10 down, whereupon the queued oligonucleotide translocase 20,20b arrests the motion of the peptide-oligonucleotide complex 10, and the translocation begins again, having, in the depicted example, re-wound by approximately 18 amino acids. Hence, in embodiments, the oligonucleotide translocase 20 may be provided at a concentration sufficient to provide multi-loading of the peptide-oligonucleotide complex 10 by the oligonucleotide translocase.

In the depicted embodiment, the peptide 11 has a first peptide end and a second peptide end. The first peptide end is linked to the oligonucleotide 13, especially via the linker 12. The second peptide end is linked to a negatively charged element 15, especially via a second linker 14. In further embodiments, the negatively charged element may be selected from the group comprising a second oligonucleotide, a charged peptide, and a charged non-peptide chemical species.

Hence, due to an imposed potential difference, the peptide-oligonucleotide complex 10 may start moving from the cis side 31 of the nanopore to the trans side 32 of the nanopore. In embodiments, a complementary oligonucleotide 40 may be sheared of as the oligonucleotide 13 enters the nanopore 50. Once the oligonucleotide translocase 20 reaches the translocase position 52, especially once the complementary oligonucleotide 40 is fully sheared off, the oligonucleotide translocase 20 may start processing the oligonucleotide 13, thereby translocating the peptide-oligonucleotide complex 10 from the trans side 32 to the cis side 31. Once the oligonucleotide translocase 20 reaches the linker 12 (or the peptide 11), the oligonucleotide translocase 20 may dissociate, thereby allowing the peptide-oligonucleotide complex 10 to move towards the trans side 32 (due to the imposed voltage) as it is no longer anchored by the oligonucleotide translocase 20. In further embodiments, a queued oligonucleotide translocase 20,20b may arrest the motion of the peptide-oligonucleotide complex 10, *i.e.,* it may anchor the peptide-oligonucleotide complex 10 again. In particular, the queued oligonucleotide translocase 20,20b may start processing the oligonucleotide 13, thereby again translocating the peptide-oligonucleotide complex 10 from the trans side 32 to the cis side 31. Hence, the same (part of the) peptide may pass the constriction 51 multiple times, and may thereby, for example, modulate a measurable current signal through the constriction multiple times.

### Example 2:

Example 2 relates to the same peptide-oligonucleotide complex as example 1.

Fig. 5 schematically depicts experimental data obtained with an embodiment of the analysis method, wherein the analysis method comprises an embodiment of the method 100 wherein the oligonucleotide translocase 20 is provided at a concentration sufficient to provide multi-loading of the peptide-oligonucleotide complex 10 by the oligonucleotide translocase. In particular, the oligonucleotide translocase was provided at a concentration of 300 nM.

In particular, Fig. 5 depicts the translocation-related signal, especially the electrical current signal: the observed ion current (in pA) over time t in seconds. As can be seen by the repeated structure with an about 4 seconds median duration from about the 300 second mark till the end of the event, multi-loading of the peptide-oligonucleotide complex 10 occurs, allowing for multiple reading of the same (part of the) peptide.

### Example 3:

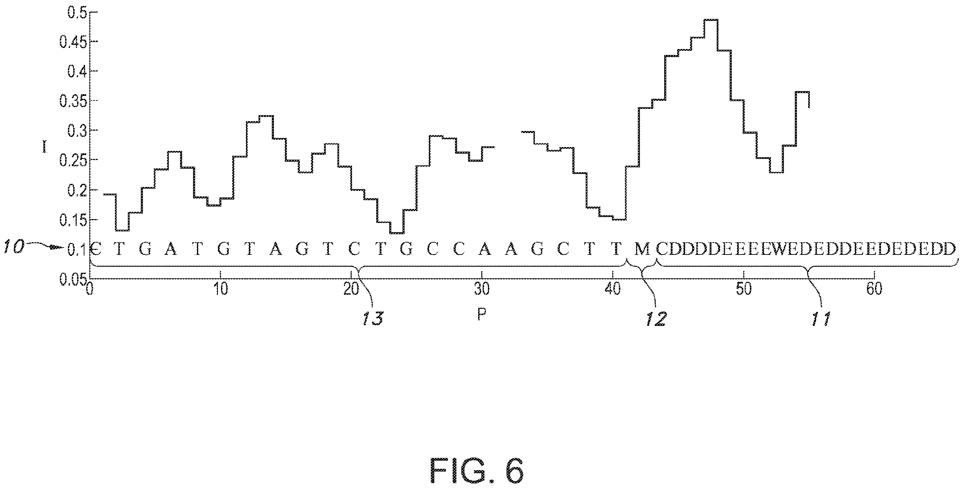

Fig. 6 schematically depicts experimental data obtained using an embodiment of the analysis method. The experiment was performed as outlined above but with a different peptide-oligonucleotide complex. Specifically, the peptide-oligonucleotide complex 10 comprised: GCTGATGTAGTCTGCCAAGCTT-M-CDDDDEEEEWEDEDDEEDEDEDD, wherein the oligonucleotide 13 comprises GCTGATGTAGTCTGCCAAGCTT, wherein the peptide 11 comprises CDDDDEEEEWEDEDDEEDEDEDD, and wherein M is the linker 12, and wherein the linker 12 is provided via maleimide-thiol chemistry. The experimentally measured values are aligned with the corresponding nucleotides and amino acids of the peptide oligonucleotide complex 10. In particular, a clear low-level blockage can be observed at the position of the tryptophan (W), which may be expected as W is much larger than the other amino acids.

The term "plurality" refers to two or more. Furthermore, the terms "a plurality of" and "a number of" may be used interchangeably.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. Moreover, the terms "about" and "approximately" may also relate to 90% or higher, such as 95% or higher, especially 99% or higher, even more especially 99.5% or higher, including 100%. For numerical values it is to be understood that the terms "substantially", "essentially", "about", and "approximately" may also relate to the range of 90% - 110%, such as 95%-105%, especially 99%-101% of the values(s) it refers to.

The term "comprise" includes also embodiments wherein the term "comprises" means "consists of'.

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

The term "further embodiments" and similar terms may refer to embodiments comprising the features of the previously discussed embodiments, but may also refer to alternative embodiments.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", "include", "including", "contain", "containing" and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings. Moreover, if a method or an embodiment of the method is described being executed in a device, apparatus, or system, it will be understood that the device, apparatus, or system is suitable for or configured for (executing) the method or the embodiment of the method respectively.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

The following are aspects of the invention:
1. A method (100) for translocation of a peptide (11) through a nanopore (50), wherein the method (100) comprises translocating the peptide (11) in the presence of an oligonucleotide translocase (20), wherein the peptide (11) is comprised by a peptide-oligonucleotide complex (10) wherein the peptide (11) is linked to an oligonucleotide (13), wherein the oligonucleotide translocase (20) is associated to the oligonucleotide (13) during at least part of the translocation.
2. The method (100) according to aspect 1, wherein the oligonucleotide (13) comprises a DNA oligonucleotide, and wherein the oligonucleotide translocase (20) comprises an enzyme selected from the group comprising a polymerase and a helicase.
3. The method (100) according to any one of the preceding aspects, wherein the method comprises providing the peptide (11) and the oligonucleotide translocase (20) to a cis side (31) of the nanopore (50), wherein the nanopore (50) is provided by a nanopore protein (55), wherein the nanopore protein (55) comprises MspA or a mutant thereof, and wherein the oligonucleotide translocase (20) comprises Hel308 or a mutant thereof.
4. The method (100) according to any one of the preceding aspects, wherein the method comprises providing the oligonucleotide translocase (20) at a concentration sufficient to provide multi-loading of the peptide-oligonucleotide complex (10) by the oligonucleotide translocase.
5. The method (100) according to any one of the preceding aspects, wherein the peptide (11) has a first peptide end and a second peptide end, wherein the first peptide end is linked to the oligonucleotide (13), and wherein the second peptide end is linked to a negatively charged element (15), wherein the negatively charged element is selected from the group comprising a second oligonucleotide, a charged peptide, and a charged non-peptide chemical species.
6. The method (100) according to any one of the preceding aspects, wherein the nanopore (50) comprises a constriction (51), wherein the constriction (51) has a circular equivalent diameter d_{c} selected from the range of 0.5 - 3 nm, and wherein the oligonucleotide translocase (20) associates with the peptide-oligonucleotide complex (10) at an anchor point (21), wherein a distance di between the constriction (51) and the anchor point (21) is at least 3 nm during at least part of the translocation.
7. The method (100) according to any one of the preceding aspects, wherein the nanopore (50) is comprised by a membrane (30), wherein the method (100) comprises providing a complementary oligonucleotide (40) at least partially complementary to the oligonucleotide (13), wherein the complementary oligonucleotide (40) is linked to a tag (42), wherein the tag (42) is configured to associate with the membrane (30).
8. The method (100) according to any one of the preceding aspects, wherein the oligonucleotide translocase (20) is selected from the group comprising non-nucleolytic enzymes.
9. The method (100) according to any one of the preceding aspects, wherein the method (100) comprises a complex-formation step, wherein the complex-formation step comprises linking the peptide (11) and the oligonucleotide (13) thereby providing the peptide-oligonucleotide complex (10).
10. The method (100) according to any one of the preceding aspects, wherein the method (100) comprises imposing a potential difference over the nanopore (50).
11. The method (100) according to aspect 11, wherein the potential difference is selected from the range of 10 - 400 mV, and wherein the method (100) comprises varying the potential difference between two consecutive steps of the oligonucleotide translocase along the oligonucleotide.
12. An analysis method for analyzing a peptide (11), wherein the analysis method comprises the method (100) according to any one of the preceding aspects, and wherein the analysis method comprises sensing a translocation related signal during the translocation.
13. The analysis method according to aspect 12, comprising: imposing the potential difference over the nanopore (50) according to any one of the preceding aspects 10-11, wherein the analysis method comprises measuring an electrical current through the nanopore (50) and providing an electrical current signal, wherein the translocation-related signal comprises the electrical current signal.
14. The analysis method according to any one of the preceding aspects, wherein the analysis method further comprises an optical read-out of the translocation of the peptide through the nanopore and providing an optical read-out related signal, wherein the translocation-related signal comprises the optical read-out related signal.
15. The analysis method according to any one of the preceding aspects 12-14, wherein the analysis method further comprises a characterization stage, wherein the characterization stage comprises characterizing the peptide (11) based on the translocation-related signal.
16. The analysis method according to aspect 15, wherein the characterization stage comprises an identification stage, wherein the identification stage comprises identifying one or more amino acids in the peptide (11) based on the translocation-related signal.
17. Use of an oligonucleotide translocase (20) to translocate a peptide-oligonucleotide complex (10) through a nanopore (50).
18. Use according to aspect 17, wherein the oligonucleotide translocase (20) comprises Hel308 or a mutant thereof.
19. Use according to any one of the preceding aspects 17-18, wherein the nanopore (50) is provided by a nanopore protein (55), wherein the nanopore protein (55) comprises MspA or a mutant thereof.
20. A kit of parts comprising an oligonucleotide translocase, a buffer comprising NTP, an oligonucleotide, and a linker suitable to link the oligonucleotide to a peptide.

## Claims

1. A method (100) for translocation of a peptide (11) through a nanopore (50),
wherein the peptide (11) is comprised by a peptide-oligonucleotide complex (10) wherein the peptide (11) is linked to an oligonucleotide (13),
wherein the method comprises:
translocating the peptide (11) through the nanopore (50) using a first molecule of an oligonucleotide translocase (20, 20a) associated to the oligonucleotide (13) during at least part of the translocation;
allowing the first molecule of the oligonucleotide translocase (20, 20a) to dissociate from the peptide-oligonucleotide complex (10); and
translocating the peptide (11) through the nanopore (50) using a second molecule of the oligonucleotide translocase (20, 20b).

2. The method (100) according to claim 1, wherein the second molecule of the oligonucleotide translocase (20, 20b) repeats the translocation of the peptide through the nanopore (50) by the first molecule of the oligonucleotide translocase (20, 20a).

3. The method (100) according to claim 1 or claim 2, comprising at least partially translocating the same peptide (11) through the nanopore (50) a plurality of times.

4. The method (100) according to any one of the preceding claims, comprising associating the second molecule of the oligonucleotide translocase (20, 20b) with the peptide-oligonucleotide complex (10) after the first molecule of the oligonucleotide translocase (20, 20a) has dissociated from the peptide-oligonucleotide complex (10).

5. The method (100) according to any one of the preceding claims, comprising multi-loading the peptide-oligonucleotide complex (10) with a plurality of molecules of the oligonucleotide translocase.

6. The method (100) according to any one of the preceding claims, wherein the method comprises providing the oligonucleotide translocase (20) at a concentration sufficient to provide multi-loading of the peptide-oligonucleotide complex (10) by the oligonucleotide translocase; preferably at a concentration of at least 100 nM, more preferably at least 300 nM.

7. The method (100) according to claim 6, wherein said multi-loading of the peptide-oligonucleotide complex (10) allows for multiple reading of the same peptide or part thereof.

8. The method (100) according to claim 7, wherein the peptide-oligonucleotide complex (10) is translocated through the nanopore (5) at least twice; at least thrice; at least four times or at least five times.

9. The method (100) according to any one of the preceding claims, wherein the method (100) comprises imposing a potential difference over the nanopore (50).

10. The method (100) according to claim 9, comprising varying the potential difference imposed over the nanopore (50) during said method, such that the peptide-oligonucleotide complex (10) is moved back and forth with respect to a constriction of the nanopore (50) in a continuous fashion during said method.

11. The method (100) according to any one of the preceding claims, wherein the peptide (11) has a first peptide end and a second peptide end, wherein the first peptide end is linked to the oligonucleotide (13), and wherein the second peptide end is linked to a negatively charged element (15), wherein the negatively charged element is selected from the group comprising a second oligonucleotide, a charged peptide, and a charged non-peptide chemical species; preferably from a second oligonucleotide and a charged peptide.

12. The method (100) according to claim 11, wherein the negatively charged element comprises a second oligonucleotide.

13. The method (100) according to claim 11, wherein the negatively charged element is attached to the peptide via a linker.

14. The method (100) according to any one of the preceding claims, wherein the oligonucleotide (13) comprises a DNA oligonucleotide.

15. The method (100) according to any one of the preceding claims, wherein the nanopore (50) is comprised by or formed by a nanopore protein;
preferably wherein the nanopore protein is selected from the group comprising MspA, CgG, FraC, and ClyA or a mutant thereof; more preferably MspA or a mutant thereof.

16. The method (100) according to any one of the preceding claims, wherein the oligonucleotide translocase (20, 20a, 20b) is a DNA translocase selected from the group comprising a polymerase and a helicase.

17. The method (100) according to any one of the preceding claims, wherein the oligonucleotide translocase (20, 20a, 20b) is Hel308 helicase, Phi29 polymerase, or a mutant thereof; preferably Hel308 or a mutant thereof.

18. An analysis method for analyzing a peptide (11), wherein the analysis method comprises the method (100) according to any one of the preceding claims, and wherein the analysis method comprises sensing a translocation related signal during the translocation.

19. The analysis method according to claim 18, comprising:
- imposing a potential difference over the nanopore (50), measuring an electrical current through the nanopore (50) and providing an electrical current signal, wherein the translocation-related signal comprises the electrical current signal; and/or
- an optical read-out of the translocation of the peptide through the nanopore and providing an optical read-out related signal, wherein the translocation-related signal comprises the optical read-out related signal.

20. The analysis method according to claim 18 or 19, wherein the analysis method further comprises a characterization stage, wherein the characterization stage comprises characterizing the peptide (11) based on the translocation-related signal; and optionally wherein the characterization stage comprises an identification stage, wherein the identification stage comprises identifying one or more amino acids in the peptide (11) based on the translocation-related signal.

21. Use of a first molecule of an oligonucleotide translocase (20, 20a) and a second molecule of the oligonucleotide translocase (20, 20b) to at least partially translocate a peptide-oligonucleotide complex (10) comprising a peptide (11) linked to an oligonucleotide (13) a plurality of times through a nanopore (50).
